# EUROPEAN PATENT APPLICATION

(11) **EP 4 666 949 A1**
(43) Date of publication of application: **24.12.2025**
(21) Application number: 24183829.1
(22) Date of filing: 21.06.2024
(51) Int. Cl.: A61B 6/03, A61B 5/00, A61B 6/00

(54) **AUTO TRIGGER OF MEDICAL IMAGING BY DIAPHRAGM STATE**

(71) Applicant: Siemens Healthineers AG, 91301 Forchheim (DE)
(72) Inventor: SUNIL, M K, 560100 Bangalore Karnataka (IN); SALINI, S., 560100 Bangalore Karnataka (IN)
(74) Representative: HKW Intellectual Property PartG mbB

(57) **Abstract**

The present invention relates to a system for acquiring medical images for providing an auto trigger of medical imaging by a diaphragm state, a method for using said system and a computer program product.

A system (1) for acquiring a medical image of a patient (2) comprising: a medical imaging unit (3) for acquiring the medical image; at least one sensor (8 - 13) for acquiring sensor data (SD) of the patient (2); an input unit (17) for receiving the sensor data (SD); and a control unit (19) for initiating the acquiring of the medical image based on the received sensor data (SD).

The system can automatically initiate the acquiring of the medical image based on the determined diaphragm state of the patient. That can render respective postprocessing algorithms unnecessary and, thus, can avoid the usage of models and assumptions.

## Description

The present invention relates to a system for acquiring medical images for providing an auto trigger of medical imaging by a diaphragm state, a method for using said system and a computer program product.

During a medical examination with the objective to take medical images of a patient, the patient is position within and/or in vicinity of a medical imaging unit. Independent of the grammatical term usage, individuals with male, female or other gender identities are included within the term. The medical imaging unit can be, in particular, an x-ray scanner, a mammography scanner, a computed tomography (CT) scanner, and/or the like. Said medical imaging unit is capable of providing an illustration of the organs within the patient, thus enabling enhanced diagnostic means like detecting organ anomalies and/or a possible grow of nodes within the organs. In order to detect anomalies of the organs and/or the growth of nodes within the organs, a number of medical images of the patient are compared with one another, which are taken at different times. In particular, a current medical image is compared with a previous image.

Nonetheless, the organs are situated within the torso of the patient. Thus, the organs of the patient both move and change their geometry depending on a diaphragm state of the patient. The diaphragm state corresponds to a state of the lungs of the patient, in particular whether the lungs are in an inhaled state, an exhaled state, or a state in between. That is due to the fact that the diaphragm, which is the muscle responsible for an inflation and a deflation of the lungs, influences other parts within the torso of the patient as well. Due to internal knowledge of the applicant, the diaphragm state can vary within the number of medical images of the patient which are about to be compared. In consequence, the comparison of the number of medical images may provide a superposition of the influence of a possibly variant diaphragm state and a variation of the organs itself, which may be an indicator for an organ anomaly or a node growth.

According to internal knowledge, said superposition can be addressed by a respective postprocessing of the medical images, wherein a non-rigid registration algorithm and/or a deformable registration algorithm is utilized to artificially manipulate the data within the medical images by "undoing" the effect of the inflation or deflation of the lungs. Nonetheless, the usage of said algorithm is based on a number of models and assumptions. Hence, the degree of uncertainty within the medical image is increased, threatening the precision or even correctness of a diagnosis based on the medical images.

In addition, it is not possible to maintain a specific diaphragm state for all medical images due to physical and/or psychological effects. For example, the patient may experience a panic attack or unconsciousness.

Therefore, it is one object of the present invention to improve the acquiring of medical images.

According to a first aspect, a system for acquiring a medical image of a patient is suggested, the system comprising
a medical imaging unit for acquiring the medical image;
at least one sensor for acquiring sensor data of the patient;
an input unit for receiving the sensor data; and
a control unit for initiating the acquiring of the medical image based on the received sensor data.

The medical imaging unit can be a CT scanner, an x-ray scanner, a mammography scanner, and/or another device configured for taking medical images. The medical imaging unit may comprise a gantry which can have an X-ray source and X-ray detectors which are both rotating within the gantry. The gantry can surround a tube, through which a bed can be moved. The bed can be located on top of a table. The bed can be configured to be movable with respect to an axial direction. The patient can be positioned on the bed in a lying manner.

The system further may further comprise a plurality of sensors. Yet, preferably, the system comprises exactly one sensor for acquiring sensor data of the patient. Hereby, the sensor data may be acquired without any active action of the patient and/or a technician using the system. In particular, pressing a mechanical and/or electronical button or the like may be unrequired in order to do so. In this context, the term "sensor data" means signals or at least one signal from the at least one sensor. The at least one sensor can be configured to acquire data of the patient, representing a physical condition of the patient. The at least one sensor can be attached to the patient and/or be in vicinity of the patient.

The system further comprises the input unit for receiving the sensor data. In particular, the input unit can be configured to convert the sensor data into a computer-readable value or values. The input unit can be configured as a hardware interface for a computer device. In case the system comprises a plurality of sensors, the sensor data of each of the plurality of sensors can be received by the input unit and converted into computer-readable values.

The control unit can be designed using electronical components, in particular, using integrated circuits. Yet, preferably, the control unit can be designed as a computer program product. The control unit can be configured to derive a current diaphragm state of the patient based on the received sensor data. Hence, the control unit can be configured to determine whether at a current time when the sensor data are acquired, the lung of the patient is exhaled, inhaled, or at a state in between. The control unit can further be configured to automatically initiate the acquiring of the medical image based on the determined current diaphragm state. Hence, based on the determined current diaphragm state, the medical imaging unit can be initiated or triggered to automatically capture the medical image. In particular, the acquiring of the medical image can be initiated at the moment in time, when the current diaphragm state of the patient corresponds to a desired diaphragm state. The sensor data and the hereby determined current diaphragm state can thus be used in the manner of an event trigger to initiate the acquiring of the medical image. Alternatively, or additionally, the technician can be informed about the determined current diaphragm state. It is feasible to inform the technician using an acoustical and/or visual indicator.

The diaphragm state corresponds to a state of the lungs of the patient, in particular whether the lungs are in an inhaled state, an exhaled state, or a state in between. That is due to the fact that the diaphragm, which is the muscle responsible for an inflation and a deflation of the lungs, influences other parts within the torso of the patient as well.

In an advantageous manner, the system can automatically initiate the acquiring of the medical image based on the determined current diaphragm state of the patient. That can render respective postprocessing algorithms unnecessary and, thus, can avoid the usage of models and assumptions. Eventually, the diagnosis according to the medical image is both rendered easier and more reliable.

According to an embodiment, the control unit is configured to initiate the acquiring of the medical image in case a current diaphragm state of the patient corresponds to a previous diaphragm state of the patient.

The acquiring of the medical image can be automatically initiated at the moment in time, when the current diaphragm state of the patient corresponds to the previous diaphragm state, wherein the previous diaphragm state corresponds to the diaphragm state of the patient at the time, when a previous medical image has been taken. For example, in case the previous medical image shows the lung in an exhaled state, for the current examination, the system can automatically acquire the current medical image at that moment when the lung is in an exhaled state as well. Hence, the system is configured to continuously acquire sensor data of the at least one sensor and to determine the current diaphragm state until the current diaphragm state and the previous diaphragm state are in agreement with one another. In consequence, the system can automatically enable an equal diaphragm state within all medical images of the patient.

According to a further embodiment, the at least one sensor is an electrode for detecting heartbeats of the patient.

The electrode is configured to detect electrical signals produced by muscle contractions of the patient. In case the patient is not moving, said muscle contractions represent the activity of the heart of the patient. Thus, the electrode is configured to detect the heartbeats of the patient, represented by peaks within the sensor data of the electrode. Therefore, the electrode can be attached to the skin of the patient. Alternatively, or additionally, the at least one sensor can be a camera for capturing a chest of the patient, a respiratory sensor for detecting a breathing condition of the patient, a kinetic sensor for detecting a movement of the patient, a sensor configured to measure a skin temperature and/or a humidity of the skin of the patient, and/or another sensor capable of expressing the physical behavior of the patient. The at least one sensor is compatible with the medical imaging unit. In particular, the at least one sensor is compatible with X-ray radiation and/or magnetic fields. In particular, it is free of interferences with the medical imaging unit. The at least one kinetic sensor can be used to recognize a movement of at least one body part of the patient, for example, a movement of the head, of the chest, or of another body part. The system may comprise a plurality of kinetic sensors, for example two, three, four, or five. The usage of a plurality of kinetic sensors enables the detection of a movement of the patient in a three-dimensional manner. The usage of the at least one sensor enables a precise acquiring of sensor data expressing the physical behavior of the patient.

According to a further embodiment, the control unit is configured to determine the current diaphragm state of the patient based on the detected heartbeats.

The control unit can be configured to analyze and to evaluate the detected heartbeats of the patient. A model can be used to analyze and to evaluate the heartbeats. Furthermore, the control unit can be configured to determine the current diaphragm state based on said analysis and evaluation of the heartbeats. Alternatively, or additionally, the control unit is configured to determine the current diaphragm state of the patient based on the captured movements of the patient. Hereby, photogrammetry can be used in order to determine the diaphragm state of the patient.

According to a further embodiment, the control unit is configured to determine a breathing pattern of the patient based on the detected heartbeats, wherein the control unit is configured to determine the current diaphragm state based on the breathing pattern.

In this context, the term "breathing pattern" represents a breathing behavior of the patient over time. The breathing pattern includes the inhalation and exhalation events of the lung of the patient. For example, characteristics within the breathing pattern can represent an inhalation into the lung of the patient. Hence, the breathing pattern can correspond to the location and/or the movement of the diaphragm. The control unit is configured to determine the breathing pattern by evaluating the detected heartbeats. The control unit is further configured to analyze the breathing pattern in order to determine the current diaphragm state. For example, in case the breathing pattern reaches a climax, the diaphragm state can be determined as being inhaled. In an analog way, a valley value of the breathing pattern can correspond to an exhaled diaphragm state.

According to a further embodiment, the control unit is configured to determine variations concerning a heart rate and/or variations concerning amplitudes of the heartbeats, wherein the heart rate may be derived from or may correspond to the reciprocal value of time intervals between subsequent heartbeats, and wherein the breathing pattern is determined based on said variations.

The control unit is configured to determine time intervals between subsequent heartbeats. Using that, the heart rate is the reciprocal value of the time intervals between subsequent heartbeats. The control unit is configured to determine variations between said heart rate. For example, it can be determined whether the heart rate is increasing or decreasing over time. In addition, the control unit is configured to determine the amplitudes of the heartbeats. The amplitude corresponds to the strength of the corresponding heart muscle contraction of a heartbeat. Using that, the control unit is configured to determine variations of the amplitudes. For example, it can be determined whether the amplitudes are increasing or decreasing over time. The control unit can be configured to determine the breathing pattern of the patient according to one of said variations or according to both variations. For example, an increasing amplitude can correspond to an increase within the breathing pattern.

According to a further embodiment, the at least one sensor is integrated within the medical imaging unit.

The at least one sensor can be integrated within the gantry of the medical imaging unit and/or be attached to the bed of the medical imaging unit. In particular, the at least one sensor is integrated in such way that a movement of the bed, for example relative to the gantry, does not interfere with the at least one sensor.

This way, the at least one sensor does not influence the acquiring of the medical image in a disadvantageous manner. In addition, the at least one sensor is not exposed to the patient or the technician, thus significantly reducing the risk of accidentally detaching and/or damaging the at least one sensor.

According to a further embodiment, the at least one sensor is installed above and facing the patient and/or the at least one sensor is hooked up with the patient. In particular, the at least one sensor is hooked up with a leg, an arm, and/or the chest of the patient.

The term "hooked up" in this context particularly means that the at least one sensor is attached to the skin of the patient. For example, the at least one sensor can be temporarily glued to the leg, the arm, and/or the chest of the patient.

According to a further embodiment, the diaphragm state of the patient is stored with the medical image.

The diaphragm state can be stored as an additional tag with the medical image. The diaphragm state can be stored within a file containing the medical image. Hereby, the diaphragm state can be written as a label within the medical image and/or stored as an annex to the medical image.

According to a further embodiment, the control unit is configured for performing a reverse engineering process, wherein the reverse engineering process comprises the determination of the previous diaphragm state according to the previous medical image.

The reverse engineering process may be a model which is run by the control unit. In particular, the reverse engineering process can be designed as a deep learning algorithm. The control unit can be configured to use a deep learning algorithm for performing the reverse engineering process. The deep learning algorithm can be configured as a classifier in order to determine the previous diaphragm state shown corresponding to the previous medical image. During a learning phase of the deep learning algorithm, corresponding diaphragm states are assigned to a large number of medical images. That is used as a training data set of the deep learning algorithm. Hereby, the training data set is diverse and representative of a wide range of individuals ensuring that the trained deep learning algorithm is robust and capable of correctly identifying the diaphragm state of patients over a wide range of different age groups and of different gender. Especially, in case the previous diaphragm state of a previous medical image is not stored with the medical image, performing the reverse engineering process can still provide the information about the previous diaphragm state and thus ensure an acquiring of the current medical image with the same diaphragm state. Note, that other methods to perform the reverse engineering process are feasible as well.

According to a further embodiment, the control unit is configured to determine the previous diaphragm state according to the location and/or the size of at least one organ visible within the previous medical image.

Preferably, the deep learning algorithm is configured to identify a plurality of organs within the previous medical image and to determine the geometry and/or the locations of the organs. "Identifying" in this context particularly means that the deep learning algorithm can identify human organs within the previous medical image, for example liver, lung, intestine, spine, or the like. The geometry and/or the locations of the organs can be determined by edge detection. The geometry and/or the locations of the identified organs, in particular the lung, can be used as an indicator for the respective previous diaphragm state.

According to a second aspect, a method for using a system with a medical imaging unit for acquiring a medical image of a patient is suggested. The method comprises:
acquiring sensor data of the patient by at least one sensor;
receiving the sensor data by an input unit; and
initiating the medical imaging unit based on the received sensor data by the control unit.

The system is embodied according to the first aspect or according to an embodiment of the first aspect.

According to an embodiment, the method further comprises determining a previous diaphragm state of the patient by the control unit; determining a current diaphragm state of the patient based on the received sensor data by the control unit; and comparing the current diaphragm state with the previous diaphragm state.

According to an embodiment, the determining of the previous diaphragm state of the patient is performed by storing the previous diaphragm state with the previous medical image and/or by a reverse engineering process analyzing the previous medical image.

According to a third aspect, a computer program product is proposed comprising instructions which, when the program is executed by a computer, cause the computer to carry out the above-mentioned method.

The computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

The embodiments and features described with reference to the system of the first aspect apply mutatis mutandis to the method of the second aspect.

Further possible implementations or alternative solutions of the invention also encompass combinations - that are not explicitly mentioned herein - of features described above or be-low with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a schematic perspective illustration of an embodiment of a system for acquiring medical images of a patient;
Fig. 2 shows a schematic illustration of an embodiment of sensor data acquired by an embodiment of an electrode of the system according to Fig. 1;
Fig. 3 shows a schematic illustration of an embodiment of a breathing pattern determined based on the sensor data according to Fig. 2;
Fig. 4 shows a schematic illustration of an embodiment of a temporal evolution of a current diaphragm state D determined based on the breathing pattern according to Fig. 3; and
Fig. 5 shows an embodiment of a method for using the system according to Fig. 1.

In the Figures, like reference numerals designate like or functionally equivalent elements, unless otherwise indicated.

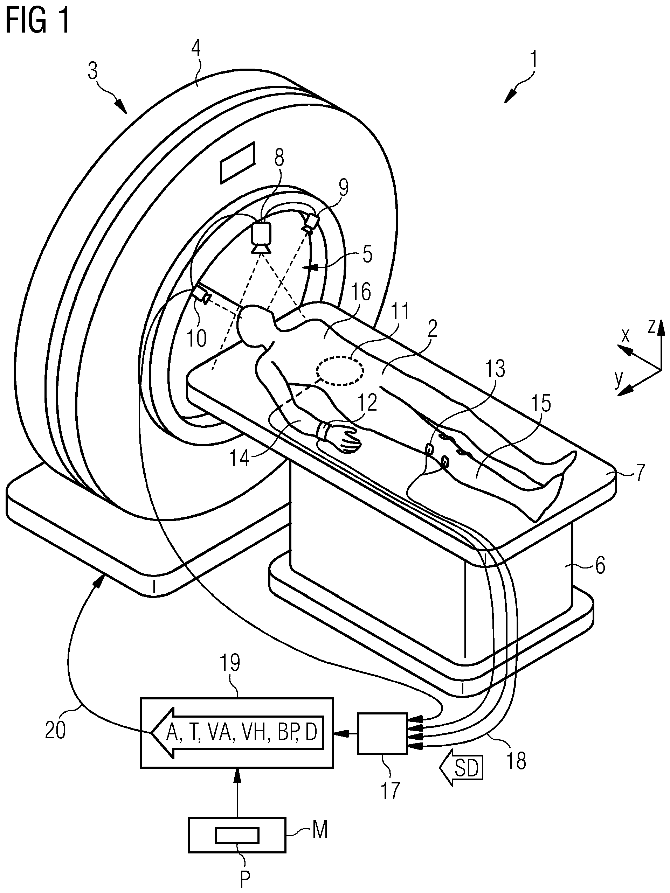

Fig. 1 shows a schematic perspective illustration of an embodiment of a system 1 for acquiring medical images of a patient 2. The system 1 comprises a medical imaging unit 3 with a gantry 4, whereby the gantry 4 contains an X-ray source and an X-ray detector (both not visible in Fig. 1). The gantry 4 surrounds a tube 5 in the shape of a narrow and long tunnel. The tube 5 extends along an axial direction x. The medical imaging unit 3 further comprises a table 6 onto which a bed 7 is attached. The bed 7 is attached to the table 6 such way that the bed 7 can be moved along the axial direction x. In particular, the bed 7 can be moved through the gantry 4.

The system 1 further comprises a plurality of sensors 8 - 13, in particular, it comprises a camera 8, two kinetic sensors 9, 10, a respiratory sensor 11, a blood pressure sensor 12, and an electrode 13. The camera 8 is integrated within the gantry 4 and on top of the bed 7 facing downwards. This way, the camera 8 is configured to capture the patient 2. The kinetic sensors 9, 10 are integrated within the gantry 4 as well at different locations inside the tube 5. This way, the kinetic sensors 9, 10 are configured to capture the patient 2 from different angles and thus enable capturing the patient 2 in a three-dimensional manner. The respiratory sensor 11 is integrated in the bed 7 underneath the patient 2. This way, the breathing status of the patient 2 can be detected by the respiratory sensor 11. The blood pressure sensor 12 is attached to an arm 14 of the patient 2 for detecting a blood pressure of the patient 2. The electrode 13 is attached to the arm 14, a leg 15, or a chest 16 of the patient 2 for detecting heartbeats H of the patient 2.

The sensors 8 - 13 are coupled with an input unit 17 via at least one sensor cable 18 of which only one is referred to with a reference sign in Fig. 1 Alternatively, or additionally, the sensors 8 - 13 can be coupled with the input unit 17 in a wireless manner. The input unit 17 converts sensor data SD of the sensor 8 - 13 into computer-readable digital data.

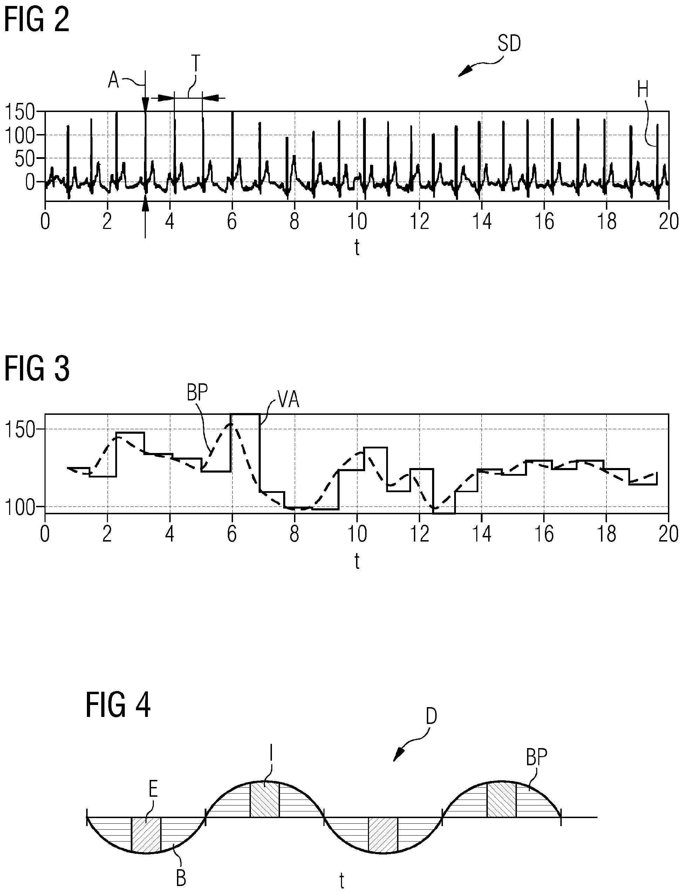

The system 1 further comprises a control unit 19 which is configured to derive a current diaphragm state D based on the sensor data SD. The control unit 19 will be described in detail below. Said current diaphragm state D corresponds to the current state of the lung of the patient 2. The current diaphragm state D can express one of the values "inhaled state" I, "exhaled state" E, and "in between state" B (see also Fig. 4). Note that there can exist other values to the current diaphragm state D as well.

The control unit 19 is configured to determine a previous diaphragm state P corresponding to a previous medical image M. The previous diaphragm state P can be stored with the previous medical image M. This way, the control unit 19 is configured to load the previous diaphragm state P from the previous medical image M. In case no information concerning the previous diaphragm state P is available, the control unit 19 is configured to perform a reverse engineering process. The reverse engineering process is a model which is run by the control unit 19. In particular, the reverse engineering process can be designed as a deep learning algorithm. Hereby, the control unit 19 is configured to identify a plurality of organs within the previous medical image M and to determine the geometry and/or the locations of the organs. "Identifying" in that context means that the deep learning algorithm can identify human organs within the previous medical image M, for example liver, lung, intestine, spine, or the like. The geometry and/or the locations of the organs is determined by edge detection. The geometry and/or the locations of the identified organs, in particular the lung, is used as an indicator for the respective previous diaphragm state P.

The control unit 19 is configured to compare the previous diaphragm state P with the current diaphragm state D. If they are not in agreement with one another, the control unit 19 is configured to further acquire sensor data SD and to determine the corresponding current diaphragm state D. If they are identical, the control unit 19 is configured to automatically initiate the medical imaging unit 3 to acquire a medical image of the patient 2, for example via an output cable 20 or via a wireless connection with the medical imaging unit 3. The control unit 19 is further configured to inform a technician (not visible in the figures) using the medical imaging unit 3 about the determined current diaphragm state D, possibly by an acoustic and/or a visual indication. The control unit 19 can be part of a computer program product for controlling the medical imaging unit 3.

Fig. 2 shows a schematic illustration of an embodiment of sensor data SD acquired by the electrode 13. The sensor data SD comprise electrical signals produced by muscle contractions of the patient 2. In case the patient 2 is not moving, said muscle contractions represent the activity of the heart of the patient 2. Thus, the sensor data SD of the electrode 13 show heartbeats H of the patient 2, represented as peaks within the sensor data SD. Note, that in Fig. 2, only one peak is referred to with a reference number.

The control unit 19 is configured to determine time intervals T between subsequent heartbeats H as well as an amplitude A of the heartbeats H, of which each only one is assigned with a reference number in Fig. 2. The amplitude A corresponds to the strength of the corresponding heart muscle contraction. It can be seen that over time, the amplitude A shows variations. Using the time intervals T, a heart rate is derived from or may correspond to the reciprocal value of the time intervals T.

Fig. 3 shows a schematic illustration of an embodiment of a breathing pattern BP determined based on the sensor data SD as described above. The control unit 19 is configured to determine variations VH of the heart rate over time. Additionally, the control unit 19 is configured to determine variations VA of the amplitudes A over time. An increase of the amplitudes A during a time interval results in an upwards-directed step of the variations VA of the amplitudes A within that time interval. The control unit 19 is configured to determine the breathing pattern BP of the patient 2 according to the variations VA of the amplitudes A and/or according to the variations VH of the heart rate. Hereby, an increase regarding at least one of said variations VA, VH can correspond to an increase within the breathing pattern BP.

Fig. 4 shows a schematic illustration of an embodiment of a temporal evolution of the current diaphragm state D as determined based on the breathing pattern BP described above. The control unit 19 is configured to determine the current diaphragm state D based on the breathing pattern BP. For example, in case the breathing pattern BP reaches a climax, the current diaphragm state D can be determined as "inhaled state" I. In an analog way, a valley value of the breathing pattern BP corresponds to the diaphragm state D being "exhaled state" E.

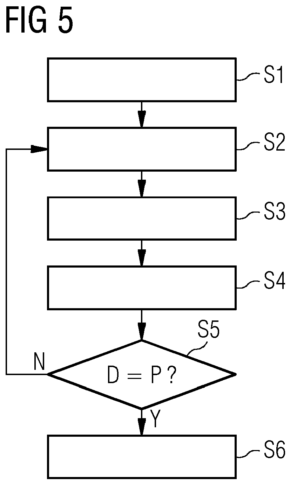

Fig. 5 shows an embodiment of a method for using the system 1 as described above. In a first step S 1, the previous diaphragm state P is determined by the control unit 19 based on a reverse engineering process of the previous medical image M or by reading the previous diaphragm state P stored with the previous medical image M. In a second step S2, the sensor data SD of the patient 2 are acquired by the at least one sensor 8 - 13. In a third step S3, the sensor data SD are received by an input unit 17. In a fourth step S4, the current diaphragm state D is determined by the control unit 19 based on the received sensor data SD. In a fifth step S5, the determined current diaphragm state D and the determined previous diaphragm state P are compared with one another. In case they match with one another, the medical imaging unit 3 is initiated to acquire the medical image by the control unit 19 in a sixth step S6. In case the current diaphragm state D and the previous diaphragm state P do not match, the second step S2 is repeated.

Although the present invention has been described in accordance with preferred embodiments, it is obvious for the person skilled in the art that modifications are possible in all embodiments.

## Claims

1. A system (1) for acquiring a medical image of a patient (2) comprising:
a medical imaging unit (3) for acquiring the medical image;
at least one sensor (8 - 13) for acquiring sensor data (SD) of the patient (2);
an input unit (17) for receiving the sensor data (SD); and
a control unit (19) for initiating the acquiring of the medical image based on the received sensor data (SD).

2. The system according to claim 1, wherein the control unit (19) is configured to initiate the acquiring of the medical image in case a current diaphragm state (D) of the patient (2) corresponds to a previous diaphragm state (P) of the patient (2).

3. The system according to claim 2, wherein the at least one sensor (8 - 13) is an electrode (13) for detecting heartbeats (H) of the patient (2).

4. The system according to claim 3, wherein the control unit (19) is configured to determine the current diaphragm state (D) of the patient (2) based on the detected heartbeats (H).

5. The system according to claim 4, wherein the control unit (19) is configured to determine a breathing pattern (BP) of the patient (2) based on the detected heartbeats (H), and wherein the control unit (19) is configured to determine the current diaphragm state (D) based on the breathing pattern (BP).

6. The system according to claim 5, wherein the control unit (19) is configured to determine variations (VH) concerning a heart rate and/or variations (VA) concerning amplitudes (A) of the heartbeats (H), wherein the heart rate is derived from or corresponds to the reciprocal value of time intervals (T) between subsequent heartbeats (H), and wherein the breathing pattern (BP) is determined based on said variations (VH, VA).

7. The system according to one of claims 2 to 6, wherein the at least one sensor (8 - 13) is integrated within the medical imaging unit (3).

8. The system according to claim 7, wherein the at least one sensor (8 - 13) is installed above and facing the patient (2) and/or the at least one sensor (8 - 13) is hooked up with the patient (2), in particular, the at least one sensor (8 - 13) is hooked up with a leg (15), an arm (14), and/or a chest (16) of the patient (2).

9. The system according to one of claims 2 to 8, wherein the previous diaphragm state (P) of the patient (2) is stored with the previous medical image (M).

10. The system according to one of claims 2 to 9, wherein the control unit (19) is configured for performing a reverse engineering process, wherein the reverse engineering process comprises the determination of the previous diaphragm state (P) according to the previous medical image (M).

11. The system according to claim 10, wherein the control unit (19) is configured to determine the previous diaphragm state (P) according to the location and/or the size of at least one organ visible within the previous medical image (M).

12. A method for using a system (1) with a medical imaging unit (3) for acquiring a medical image of a patient (2); the method comprising:
acquiring (S2) sensor data (SD) of the patient (2) by at least one sensor (8 - 13);
receiving (S3) the sensor data (SD) by an input unit (17); and
initiating (S6) the acquiring of the medical image based on the received sensor data (SD) by a control unit (19).

13. The method according to claim 12, further comprising
determining (S1) a previous diaphragm state (P) of the patient (2) by the control unit (19);
determining (S4) a current diaphragm state (D) of the patient (2) based on the received sensor data (SD) by the control unit (19); and
comparing (S5) the current diaphragm state (D) with the previous diaphragm state (P).

14. The method according to claim 13, wherein the determining (S1) of the previous diaphragm state (P) of the patient (2) is performed by storing the previous diaphragm state (P) with a previous medical image (M) and/or by a reverse engineering process analyzing the previous medical image (M).

15. A computer program product comprising instructions which, when the program is executed by a computer, cause the computer to carry out the method of one of claims 12 or 14.
